# EUROPEAN PATENT APPLICATION

(11) **EP 1 462 118 A1**
(43) Date of publication of application: **29.09.2004**
(21) Application number: 04251702.9
(22) Date of filing: 24.03.2004
(51) Int. Cl.: A61K 49/00

(54) **Compositions including a visual marker and method of use thereof**

(30) Priority: 26.03.2003 US 397392
(71) Applicant: Pediamed Pharmaceuticals, Inc., Florence, Kentucky 41042 (US)
(72) Inventor: Heasley, Ralph A., Union Kentucky 41091 (US); Gonzales, Gilbert R., New York New York 10021 (US)
(74) Representative: Findlay, Alice Rosemary

(57) **Abstract**

Compositions and method including a visual marker for monitoring patient compliance with a medication regimen. The marker is formulated with a rapid dissolve layer and adhered to, or ingested with, an orally administrable medicament. Upon ingestion, the marker is released causing a detectable marker-induced property in the oral and/or pharyngeal cavity of a subject. By examining the patient's oral and/or pharyngeal cavity and articles associated with the patient, one can determine whether the medicament has been ingested based upon the presence or absence of the property. The composition is also useful for tracking the location of an un-ingested medicament.

## Description

The present invention relates generally to monitoring ingestion of a compound, and more particularly to compositions and methods used for monitoring a patient to determine compliance by the patient with a medication regimen.

The term "compliance" in the practice of medicine, and specifically in pharmacotherapy, is defined as the "extent to which the patient's behavior coincides with the clinical prescription" (Litt, I.F. and Chuskey, W.R., *Compliance with medical regimens during adolescence,* Pediatric Clin North Am, 27:3, 1980).

When selecting a medication for a specific patient, many factors are considered, including the medication's efficacy profile, safety profile, route of administration, price, and the compliance of the patient in taking the medication. If a medication must be taken more than once a day, compliance becomes an important factor in selecting a drug because the pharmacologic efficacy of the medication will be more adversely affected if the medication is not taken as directed. The problem of noncompliance with a prescribed regimen has become so serious that, in response to such a problem, the pharmaceutical industry has developed long-acting forms of many medications.

The problems with noncompliance are particularly pronounced among certain groups of patients. These groups include: (1) pediatric patients, particularly those in child care centers or schools where medications are to be delivered by caregivers or teachers; (2) geriatric patients, whose caregivers are present only intermittently; (3) mentally handicapped individuals, who live independently or whose caregivers are present only intermittently; and (4) disease or disorder specific groups, including patients suffering from alcohol dependence, drug dependence, seizures, certain psychiatric conditions, cardiovascular disease, hypertension, or other conditions.

Accordingly, noncompliance is a problem that is widespread in society. Research has shown that patients only ingest half of the medication that is actually prescribed by physicians (Haynes, R.B., Taylor, D.W., and Sackett, D.L., Compliance in Health Care, Johns Hopkins University Press, Baltimore, 1979). Other studies have shown that up to 93% of medication regimens are not followed as prescribed (Greenberg, R.N., *Overview of patient compliance with medication dosing: A literature review,* Clin Ther, 6:592-599, 1984). The proportion of those that do not ingest their prescribed medication is greatest when social and cultural barriers, such as a language difficulty, exist, or when a decline in cognitive understanding, such as memory loss, interferes with carrying out instructions. Also, compliance varies with the illness that is treated, the degree of distress associated with symptoms, the complexity of the dosing regimen, the duration of the disease, and the extent of the adverse effects (Del Boca, F.K., Kranzler, H.R., Brown, J., and Korner, P.F., *Assessment of medication compliance in alcoholics through UV light detection of a riboflavin tracer,* Alcohol Clin Exp Res, 20(8):1412-1417, 1996; Babiker, I.E., Cooke, P.R., and Gillett, M.G., *How useful is riboflavin* as a *tracer of medication compliance?,* J Behav Med, 12:25-38, 1989).

Therefore, noncompliance is a major problem in medicine in general, and in several diseases in particular. As an example, schizophrenia is associated with a noncompliance rate of 11 % to 50% with an average of 33% (Maarjberg, K., Aagaard, J., and Vestergard, P., *Adherence to lithium prophylaxis: 1. Clinical predictors and patient's reasons for non adherence,* Pharmacopsychiatry 21:121-125, 1988; Kane, J.M. and Borenstein, M., *Compliance in long-term treatment of schizophrenia,* Psychopharmacol Bull, 21:23-27, 1985; Van Putten, T., *Why do schizophrenic patients refuse to take their drugs?,* Arch Gen Psychiatry, 31:67-72, 1974; Babiker, I.E., *Noncompliance in schizophrenia,* Psychiat Dev, 4:329-337, 1986). As a result, multiple areas of medicine have been subject to extensive and specific methodologic testing for compliance. For example, the use of riboflavin fluorescence in the urine has been used for testing compliance for various conditions and diseases. These include schizophrenia, clinical drug trials, alcohol dependence, iron deficiency, tricyclic antidepressant therapy, hypertension medication, the use of oral contraceptives in adolescents, anti-epileptic drug use, and cardiovascular diseases (Babiker, et al., *How useful is riboflavin* as a *tracer of medication compliance?,* J Behav Med, 12:25-38, 1989; Anton, *New methodologies for pharmacological treatment for alcohol dependence,* Alcohol Clin Exp Res, 20(7 Suppl): 3A-9A, 1996; Cromer, et al., *Psychosocial determinants of compliance in adolescents with iron deficiency,* Am J Dis Child, 143(1):55-58, 1989; Gilmore, et al., *A study of drug compliance, including the effect of a treatment card, in elderly patients following discharge home from hospital,* Aging (Milano), 1(2):153-158, 1989; Perel, *Compliance during tricyclic antidepressant therapy: pharmacokinetic and analytical issues,* Clin Chem, 34(5):881-887, 1988; Sullivan, et al., *Compliance among heavy* alcohol *users in clinical drug trials,* J Subst Abuse, 1(2):183-194, 1988-1989; Tinguely, et al., *Determination of compliance with riboflavin in an antidepressive therapy,* Arzneimittelforschung, 35(2):536-538, 1985; Durant, et al., *Influence of psychosocial factors on adolescent compliance with oral contraceptives,* J Adolesc Health Care, 5(1):1-6, 1984; Jay, et al., *Riboflavin, self-report, serum norethindrone. Comparison of their use* as *indicators of adolescent compliance with oral contraceptives,* Am J Dis Child, 138(1):70-73, 1984).

Other methods to determine medication regimen compliance include clinical observation of patients, and the analysis of their bodily excretions. One common method of monitoring patients for medication regimen compliance is clinical observation involving individual counselling and close personal supervision by physicians. For example, physicians may observe a patient for physiological signs and symptoms indicative of compliance or noncompliance. These signs and symptoms may include residual signs of illness. Alternatively, the patient may be interviewed regarding the degree of relief from the affliction. A physician might also evaluate physiological changes in the patient. Clinical observation, however, is time consuming and, therefore, expensive. Furthermore, it is dependent on the physician=s subjective opinion, and therefore is subject to potential errors.

Still other methods of obtaining compliance information include qualitative urine monitoring methods. One example is the standard laboratory procedure known as enzyme-multiplied immunoassay (EMIT). Utilizing an arbitrary cutoff value, these methods provide the clinician with a simple positive or negative indication of the possible presence or absence of a parent drug or its metabolites in a patients urine. Urine monitoring methods may also be used to provide a quantitative analysis of ingestion of medication. However, whether qualitative or quantitative, several drawbacks exist in these analytical methods.

First, these analytical methods and tests are time and labor-intensive, often requiring the use of complex equipment in the analysis, and thus are not particularly useful when the time period between medication dosages is short. Second, these methods generally require a trained technician to perform the analysis. Third, the analysis is often performed at a location remote to the site where the sample is obtained. Finally, the sample collection itself, for example, obtaining a urine sample, involves a heightened degree of intrusiveness for the patient. As a result, these methods are not amenable to a rapid, less intrusive, on-site assessment of compliance.

In an attempt to ameliorate some of the above-discussed problems of the monitoring methods of the prior art, markers have been used to determine the presence of medication in the system of a subject. For example, clinical markers or labels have been synthetically attached to active ingredients or other components of the medication. However, in order to do so, the manufacturer must alter its synthetic procedures, formulation technology, and/or manufacturing equipment to produce the final commercial medication. In addition, inclusion of markers in such a manner create extensive validation, stability testing, and other regulatory requirements before the final medication can be approved for use. Consequently, these markers have drawbacks including raising the costs of the medication, increasing the time in delivering the medication to the marketplace, and the potential for side effects from ingestion of the medication. In addition, detecting such markers for monitoring patient compliance with a medication regimen still requires that the urine or stool of a subject be examined by a trained professional. Thus, while reducing some of the time and complexity related to traditional monitoring methods, these tests are still not useful as a Home" test, still require some heightened degree of time, labor and expense, and do nothing to reduce the intrusiveness experienced by the patient.

While providing information relative to patient status and treatment compliance, the clinical monitoring methods described above have distinct drawbacks which limit their usefulness in determining compliance. Thus, it would be desirable to have a monitoring method that is rapid, simple, and inexpensive. It would also be desirable for such a test to be amenable to use as and when needed, for example at the point of care. It would be further desirable for such a test to be conveniently used by anyone, including laypersons in their own homes. It would be still further desirable for such a test to be minimally intrusive to the patient.

The present invention in one embodiment provides compositions including at least one marker present in a form and amount sufficient to cause a visually detectable marker-induced property, such as coloration. The coloration may be of at least a portion of the oral and/or pharyngeal cavity of a patient. The composition is placed on and ingested with an orally administrable medicament. Exposure of the composition to the patient's saliva upon oral ingestion of the medicament causes release of the marker. To this end, a portion of the composition is activated by saliva, such as by dissolving in the saliva for example, to release the marker in the mouth of the patient in a sufficient amount and in a distinct form to cause the marker-induced property following ingestion. For example, in one embodiment, the marker may stain a portion of the buccal membrane.

In accordance with a preferred embodiment of the present invention, the composition comprises a rapid dissolve layer formulated with one or more markers. The rapid dissolve layer may comprise a material which is soluble in saliva and releases the marker upon exposure to saliva. The marker is a visually detectable material, such as a dye, a fluorescent compound, a visually observable solid, or a combination of such materials. To this end, the marker may be colored or clear, depending upon the method of detection. In another embodiment, the composition further comprises a first layer which overlies the rapid dissolve layer. The first layer may thus serve to protect the rapid dissolve layer from degradation during non use of the composition. The first layer may also be colored to provide visual identification of the composition, as well as identification and location of the medicament on which it may be placed. For the latter purpose, a friable first layer would be useful. Further, the composition may include an adhesive to adhere the composition to the medicament. Still further, the composition may include a release liner to protect the adhesive when the composition is not adhered to a medicament. As the composition may be ingested, the first layer, the adhesive, and the release liner should be pharmaceutically acceptable materials. The composition with any or all of the components described herein may be packaged into a simple and convenient form for use. For example, the composition may be in a form, such as a web, a sheet, a strip, a dot, a tapedot, a microdot, or a multilamelar dot, that is easy to apply to the medicament by simply placing it thereon. Utilizing kis-cut technology to manufacture the compositions may be economical. Having a compliance monitoring marker separate from the medication and utilizing it as and when needed generally reduces costs and is more convenient for persons caring for the patient.

In accordance with another embodiment of the present invention, there is provided a medicament formulation including a medicament having thereon a rapid dissolve layer formulated with a marker. The medicament is intended for ingestion and, therefore, may be an orally administrable solid, such as a pill, a capsule, a chewable tablet, or a soft-gel capsule for example. As described above, the marker-containing rapid dissolve layer may be a composition easy to apply to the medicament and may include any or all of the beneficial components described herein. In one embodiment, the composition is adhered to the medicament sometime after manufacture of the medicament, such as by the manufacturer before packaging of the medicament. In another embodiment, it is adhered after packaging, such as at the point of administration to a patient, by a person so qualified.

In yet another embodiment, there is provided a kit for monitoring patient compliance with dosing of a medicament. The kit includes a medicament and a marker-containing rapid dissolve layer formulated in a composition designed to be placed on the medicament. Both the medicament and composition may be as described herein. Such a kit allows a caregiver, such as a member of the patient's family, to adhere the composition to the medicament at the point of care, and particularly, just prior to ingestion.

The present invention also provides methods of tracking medicaments, and particularly for monitoring patient ingestion of an ingestible medicament. In one embodiment, a medicament formulation, i.e., a medicament containing a compliance marker in a saliva-activated rapid dissolve layer, is provided to the patient for ingestion. Later, the patient is examined for detection for a marker-induced property to track the location of the medicament. The marker may be examined by visual inspection of the oral and/or pharyngeal cavity of the patient for monitoring ingestion by the patient. Alternatively, articles associated with the patient, such as the clothing and body parts, may be visually inspected for detecting the marker-induced property or the presence of the marker itself, and for determining the location of the medicament. Visual inspection is less intrusive than monitoring methods of the prior art.

In accordance with another embodiment of the present invention, multiple markers are utilized wherein one marker has a property distinguishable from the other markers. For example, one marker may cause a coloration of a portion of the oral and/or pharyngeal cavity for a duration greater than another marker. That is, the coloration caused by one marker may be visually detected longer than the coloration from the another marker. In that way, the presence or absence of particular colorations may be determined in order to determine a time frame in which the composition was ingested. The color of the marker may be time related such that after a period of time following ingestion, the visually apparent coloration or discoloration could evanesce leaving no significant coloration of a portion of the oral and/or pharyngeal cavity. In that way, multiple dosages could be monitored without the coloration from a previous dose significantly interfering with the next, subsequent dose.

The marker(s) may also induce properties from which they may be distinguished. Such properties include, without limitation, color, intensity, fluorescence, and combinations thereof. For example, one marker may cause a different coloration than another marker. Further, different colored markers may have different durations, such that detecting one coloration more strongly than another may indicate the length of time since the ingestion of the last dosage of the medication composition and may help establish a time frame of the last dosage. Another basis for differentiating markers is the light used for detecting the marker. For example, suitable light such as visible light, fluorescent light, ultraviolet light, infra-red light, or X-ray, may be used for visual detection. In one embodiment, one marker causes coloration detectable with visible light, while another marker causes coloration detectable with a light which causes fluorescence. As discussed above, the duration of the different markers may be adjusted so that an approximate time since the last dosage may be determined. In one embodiment of the invention, the multiple markers might all cause fluorescence with different fluorescent colorations so that detecting predominantly one coloration, rather another coloration, will give further information with respect to the compliance time frame and the most recent dosage. While the naked eye should be sufficient for detection purposes, the present invention does not preclude the use of optical instruments or other detection instrumentation.

The use of an orally ingested and visible marker provides a qualitative determination of a patient's compliance with a medication regimen. In providing for this determination rapidly, visually, and with minimal intrusion to the patient, the present invention reduces and/or eliminates various of the drawbacks of the prior art. The marker can be directly visually detected soon after oral administration of the medication and also may be detected some time later by fluorescing the marker in the oral and/or pharyngeal cavity.

The marker indicates to a primary caregiver, a medical professional, a guardian, or a family member whether the medication was orally ingested during a recent period of time. The presence of a change, such as coloration of the oral and/or pharyngeal cavity, caused by the marker is the specific information to notify the caregiver that the medication was actually ingested.

The embodiments provide convenience in monitoring compliance of ingestion of medications will also improve medication delivery. For example, the noncompliant behavior will be altered by notifying the patient that compliance is being monitored. Should noncompliance persist, that information would allow a caregiver to alter the methods of medication delivery. For example, a child in school or daycare who requires daytime dosing of an antibiotic for recurrent ear infections could be given an antibiotic containing the marker. The child could then be checked for ingestion compliance immediately after scheduled ingestion, or if necessary, several hours after the scheduled ingestion. The verification could occur, for example, by visual inspection of the oral and/or pharyngeal cavity immediately after the delivery, for example, by the provider, under natural light or several hours later, for example, by a parent, by inspection under a light which causes fluorescence.

The embodiments thus provided compositions and methods for monitoring the compliance of a patient in following a medication regimen while reducing and/or eliminating the problems associated with monitoring methods of the prior art. They reduce the time, effort, complexity, cost, and intrusiveness of prior art monitoring methods.

The preferred embodiments of the invention will now be further described in detail

The term "patient", as used herein, is intended to refer to mammals who are in need of a medication for the treatment of a disorder or disease, as well as to mammals who are merely participating as subjects of a study and not suffering from any disorder. The composition is to be placed on, or adhered to, a medicament to form a medicament formulation to be ingested by the patient. Upon ingestion and exposure of the formulation to saliva, the marker is released in the patient's oral and/or pharyngeal cavity thereby causing a marker-induced property in the patient's oral and/or pharyngeal cavity. The property may be one of color, such as a coloration which can be visually detected either directly with visible light, or indirectly through fluorescence. Related to color change, the marker should, therefore, be present in the composition in an amount and form sufficient to cause a coloration or discoloration/staining of at least a portion of the oral and/or pharyngeal cavity. For example, the buccal membrane may be marked and/or the gum, tongue, and other oral and/or pharyngeal surfaces may also be marked or stained. While the description herein describes this property with reference to a coloration of the patient's oral and/or pharyngeal cavity, the property may be other than coloration or staining of the patient's mouth.

In accordance with the principles of the present invention, the composition may be used to monitor ingestion of a medicament. The term "medicament", as used herein, is intended to refer to an orally administrable medication. By way of example, suitable orally administrable medications include a pill, a tablet, a capsule, and a soft-gel capsule. The medication has a desired medicinal or similar effect on the patient. For example, the medication may have certain medicinal properties for treating a particular disease or symptom. In accordance with another aspect of the present invention, the compliance regimen might be determined utilizing the marker-containing composition of the present invention with a placebo composition having little or no medicinal effect. In that way, before the patient ingests the medication, the particular regimen and the patient's compliance with the regimen may be determined. For example, it may not be desirable to have a patient ingest a particular medication unless that patient can be relied upon to follow or comply with the regimen prescribed by a physician.

To monitor ingestion, the inventive compositions comprise at least one marker. The term "marker", as used herein, refers to an ingestible substance which provides properties by which the presence of the marker may be detected. Marker presence in turn indicates the likelihood of medicament presence in the area in which the marker was detected. Color is but one of many marker-induced properties that may be used to detect the presence of the marker. For example, in the oral and/or pharyngeal cavity of the patient, a marker may be visually detected via color, under either a natural light or an optimal exciting light of desired wavelength to create fluorescence (generally referred to as a fluorescent light). To this end, the marker may cause a coloration or discoloration/stain on the patient, such as on a portion of the oral and/or pharyngeal cavity. This "coloration" or color property refers to an effect of the visual marker. It should be understood that the term "coloration" is not limiting in its definition, and will also encompass discoloration or staining of a portion of the observed cavity in addition to a coloration which may actually match, to a certain extent, the natural coloration of the oral and/or pharyngeal cavities. For example, the marker may be detectable from its fluorescence, and may not otherwise visually discolor or stain the observed cavity. In another example, a very noticeable staining may occur. Similarly, color patterns of markers can be used for detection purposes. As such, the term "coloration" is used broadly herein.

A marker which is both detectable using natural light and fluorescent light, provides flexibility with respect to the visual observation of a caregiver and the determination of the presence or absence of coloration caused by the marker. For example, the marker may cause a coloration which is detectable shortly after ingestion of the composition, but which may require fluorescence for detection after a certain time period has elapsed. In either case, the caregiver may simply shine light from a hand-held instrument or other source onto the region of the patient for examination thereof and visually inspect the region. Inspection may be assisted with additional instrumentation necessary to visualize and detect the marker and/or the marker-induced property.

Another property of a marker that may be used in detection is one of duration. Duration is useful for monitoring ingestion in compliance with a dosage regimen. The duration of a marker and its effects should be such that its detectable presence should generally end, or be significantly reduced, and/or otherwise change before the next dosage is due, in order to indicate to a caregiver that another dose can or should be given. Otherwise, any significant presence of the marker at the effective time for the next medication dose may make it unclear to the caregiver whether the next dose should be given. For example, medication with a half-life requiring a new dosage approximately every six hours (for example, ampicillin) should have a marker or markers that are detectable at the time of ingestion and generally for some time period after ingestion, but not significantly beyond six hours. As discussed above, the marker may be detectable for a certain time period under natural light, and then may require fluorescence for the remaining time of detection. Although, in general, the detection duration of the marker should terminate around or before the time for the next medication dosage. The detection duration may extend beyond that dosage time, albeit with a significantly reduced detectable presence. Therefore, the present invention is not limited to markers with any specific duration. Further, the markers may be tailored for a particular use. As one example, a caregiver trained in compliance determination may be able to determine the time for the next dosage, even though the marker is still detectable. As another example, the level of detection may indicate, based upon experience, that the last dosage was given a significant time preceding such that another dosage may be given.

In accordance with one aspect of the invention, duration of the marker and its prolonged detection may be achieved through various means. For example, the marker may be present in a sufficiently high concentration so as to ensure the desired duration and prolonged detection following release. Furthermore, encouraging patients to keep the medicament formulation in their mouths longer by prolonging the swallowing time, may also affect the duration of the marker and its effective detection time. For example, a medication which is tasty or sweet may encourage children, and even adults, to maintain the formulation in their mouths for a longer period of time. In another aspect of the invention, as discussed below, multiple markers might be utilized wherein each marker has a different duration. In that way, visual inspection may give the caregiver an indication of the time since the last dosage, based upon the particular marker which is predominantly detectable. The various features discussed above are applicable to situations wherein either a single marker is utilized or multiple markers are utilized.

In another embodiment of the invention, multiple markers are utilized in the composition. Multiple markers in many cases may provide greater flexibility in detection schemes and compliance determination. For example, each of the multiple markers might have different coloration or duration characteristics. More specifically, for example, multiple markers may be utilized wherein one of the markers causes a coloration of a portion of the oral and/or pharyngeal cavity for a longer time than another of the markers. A caregiver could then determine the presence or absence of colorations caused by one or more of the multiple markers to determine the time frame in which the marker-medication or marker-placebo combination was ingested. For example, one marker may have a duration which is approximately half of the time between recommended dosages, whereas the other marker may have a duration which lasts up to or around the end of the time period between dosages. By visually observing both markers in the inspected cavity, a caregiver will know that the composition was ingested relatively recently. If only the marker with the longer duration is detected, the caregiver will know that it has been at least a certain amount of time since the composition was ingested. In that way, a particular time frame since ingestion of the composition might be ascertained. The previous example discusses the use of two markers. However, a greater number or combination of markers might be utilized to determine compliance and to more accurately pinpoint the time frame since ingestion of a medication, and the possible time for a new dose.

In another embodiment of the invention, multiple markers might be utilized which have different coloration characteristics, and therefore cause different coloration of the portion of the oral and/or pharyngeal cavity at issue. Different colorations may also be utilized for more accurate compliance determination, and also for determining the time frame between ingestion and doses. For example, markers having different durations may also have different colorations. Therefore, visual detection of the different markers will give an indication to the caregiver of the different marker durations which are being observed. One coloration might be predominantly observed directly after ingestion and another coloration may be predominant after a period of time.

In another embodiment of the invention, one or more of the markers may cause coloration of the oral and/or pharyngeal cavity which is detectable with natural light, whereas another of the markers may cause a coloration which is detectable only with a light that causes fluorescence. Again, the different qualities of the marker may provide for greater flexibility in determining compliance and dosing time frames. For example, the marker causing a coloration which is detectable with natural light may have a far shorter duration, whereas a marker which is detectable through fluorescence may have a longer duration. In that way, a time frame might be determined in which the composition was ingested and when the next dose might be required based upon the way the marker is detected.

In still another embodiment of the invention, every multiple markers might cause fluorescence, but each with a different fluorescent coloration. That is, the detection of all markers may require fluorescence. However, the particular color of the fluorescence which dominates may give an indication of the marker which is being predominantly detected. By using markers which have varying durations or by formulating the markers to provide the desired duration, further information may be ascertained through visual inspection with respect to the time frame of ingestion of the composition and time frame for the next required dose in the compliance regimen. In another embodiment, the intensity of the marker detected at a defined wavelength, or the intensity over a range of wavelengths is a property used to determine ingestion of the medicament.

Different types of available markers may be used in accordance with the embodiments of the present invention. For example, in one embodiment of the invention, the marker is selected from a dye, a fluorescent compound, a visibly observable solid and a combination thereof. Dyes are known to stain for sufficient periods of time and, therefore, are suitable markers. Such dyes may include, without limitation, indigo carmine, methylene blue, tartrazine, laccaic acid, bet-carotene, FD&C blue 1, FD&C blue 2, FD&C green 3, FD&C red 3, FD&C red 40, FD&C yellow 6, riboflavin, and combinations thereof. Each dye, however, should be medically safe or approved and should provide a visually detectable effect, such as a coloration or discoloration of the oral and/or pharyngeal cavity, either under visible light or through fluorescence, as described above. Furthermore, the dye should be present in the composition in an amount and form to cause detectable coloration or discoloration of the oral and/or pharyngeal cavity.

In one embodiment of the present invention, carmine red dye is used as the marker. The carmine red dye provides a stain of a portion of the oral and/or pharyngeal cavity and is, thus, used to determine whether the medicament has been orally administered. This determination occurs by visual inspection either shortly after a scheduled ingestion or after a substantial amount of time following the scheduled ingestion. The discoloration or stain caused by the carmine red dye can be viewed either directly with the naked eye under visible white light or by fluorescing the residue of carmine red dye in the observed cavity.

Carmine red dye is approved by the U.S. Food and Drug Administration as a color additive for use in human food with no restrictions, as a color additive for use in topical drugs, and as a color additive for use in cosmetics. Carmine red dye is a red or purplish-red pigment derived from cochineal beetle shells that are crushed. In addition to being visible under natural light, commercial carmine produces a strong reddish-orange fluorescence at an exciting light wavelength of around 436 nanometers and again in the exciting light range from about 450 through 490 nanometers.

Additionally, the inventors are not aware of any reported embryotoxic effects of carmine in animal studies (Grant, et al., *Tetratogenicity and Embrotoxicity Study of Carmine of Cochineal in the Rat,* Food Chem Toxicol, 25(12):913-917, 1987). Carmine red is considered one of the most inert and safest food additives known and is used in medical procedures, as noted in several publications (Miller and Anderson, *Silent Regurgitation in day case Gynecological Patients,* Anaesthesia, 43(4):321-323, 1988; Read, et al., *Transit of a Meal through the Stomach, Small Intestine, and Colon in Normal Subjects and its Role in the Pathogenesis of Diarrhea,* Gastroenterology, 79(6);1276-1282, 1980; Higgs, et al., *Assessment of Simple Methods of Measuring Intestinal Transit* *Times in Children with Gastroenteritis,* Gut, 16(6):458-461, 1975; Hallagan, et al., *The* Safety *and Regulatory Status of Food, Drug and Cosmetics Colour Additives Exempt from Certification,* Food Chem Toxicol, 33(6):515-528, 1995; Schmidt, *ATagged" Local Anesthetic Solution for Transurethral Surgery,* Urology, 34(5):305-306, 1989; Reece, et al., *Transabdominal Needle Embryofetoscopy:* a *new technique paving the way for early fetal therapy,* Obstet. Gynecol, 84(4):305-306, 1994). Carmine red dosages up to 4,500 mg/day have been utilized. The present invention may not require such dosages, and levels in the range of approximately 100 mg/day or less may be sufficient in the applications of the present invention.

Suitable fluorescent compounds include not only the dyes mentioned above, but also non-dye staining compounds which fluoresce under the appropriate wavelength of light. These fluorescent compounds should be medically safe or approved and should provide a visually detectable coloration or discoloration of the oral and/or pharyngeal cavity through fluorescence. Examples of suitable fluorescent compounds include, without limitation, compounds disclosed in U.S. Patent No. 5,885,677, the disclosure of which is incorporated by reference herein in its entirety.

Other visibly observable solids, such as fine powders, which are not categorized as a dye or a fluorescent compound, are also suitable as markers in the present invention. These solids should be medically safe, or approved for consumption, and should persist in the ingesting patient's oral and/or pharyngeal cavity for periods of time following ingestion to enable detection thereof and determination of ingestion of the medication. Examples of suitable observable solids include, without limitation, mica, lycopodium powder, fumed silica, charcoal, carbon black, starch granules, diatomaceous earth, silicates such as magnesium aluminum silicate, aluminum oxide, and calcium carbonate.

To aid the persistence of the marker in the patient's oral and/or pharyngeal cavity, a bioadhesive, such as a mucoadhesive, may be used in conjunction with the marker(s) in the present compositions. The term "mucoadhesive", as used herein, refers generally to natural or synthetic pharmaceutically acceptable materials which are capable of sticking to a mucous membrane, resulting in adhesion of the material to the tissue for a protracted period of time. In order for a material to be a mucoadhesive, it must interact with mucus, which is a highly hydrated, viscous anionic hydrogel layer protecting the mucosa. The mucoadhesive material will act as a carrier for "sticking" the marker to the mucosal tissue in the oral and/or pharyngeal cavity of the patient for a prolonged period of time. To this end, the mucoadhesive may be chemically associated with the marker, such as a bioadhesive inherently possessing marker properties, or having a marker covalently bound thereto. The mucoadhesive should not interfere with the ability to examine or detect the marker(s). The mucoadhesive should also be medically safe or approved for consumption. Suitable mucoadhesives include, without limitation, materials comprised of pharmaceutically acceptable polymers, such as polyisobutylene and polyacrylic acid polymers, chitosan, and modified cellulose such as hydroxypropyl methylcellulose. Examples include, without limitation, Carbopol™ and polycarbophil, available from Noveon, Inc., Cleveland, OH. Additional suitable mucoadhesive materials are disclosed in Leung et al., *Polymer Structural features Contributing to Mucoadhesion II,* J. Contr. Rel., 12(3), 187-94, 1990; Ch'ng et al., *Bioadhesive Polymers* as *Platforms for Oral Controlled Release Drug Delivery II: Synthesis and Evaluation* of *Some Swelling water-insoluble Bioadhesive Polymers,* J. Pharm. Sci., 74, 229, 1985; and Guo, J-H., *Bioadhesive Polymers Buccal Patch for Buprenorphine Controlled Drug Delivery Formulation, In Vitro Adhesion and Release Properties,* Drug. Devel. Ind. Pharm., 20(18), 2809-2821, 1994, the disclosures of which are incorporated herein by reference in their entireties.

In accordance with another aspect of the invention, it is contemplated that the marker be contained in a "taggant" or a "microtaggant", suitable for pharmaceutical use, and oral administration. To this end, it is believed that microtaggants have not yet been federally approved for pharmaceutical use. However, a color coated marker may be present in a composition like that of a microtaggent, detectable in the oral and/or pharyngeal cavity of the patient by methods described herein without release from the microtaggant housing. Additional description of microtaggants is disclosed in US Patent No. 6,432,715, which disclosure is incorporated herein by reference in its entirety.

In one embodiment of the present invention, the marker(s) is formulated with a rapid dissolve layer in the composition. Thus, the marker(s) are contained in the rapid dissolve layer. The terms "contained" and "marker-containing", as used herein with reference to the relationship between the marker(s) and the rapid dissolve layer, are used broadly and generally refers to the marker(s) being present either within the rapid dissolve layer, on the rapid dissolve layer, or as an integral component of the rapid dissolve layer. The rapid dissolve layer is activated by saliva to release the marker(s) from the composition. Accordingly, upon exposure of the rapid dissolve layer to the patient's saliva, following ingestion of the medicament formulation, the marker(s) is released into the patient's oral and/or pharyngeal cavity. The rapid dissolve layer will, therefore, be comprised of materials which react with saliva so as to free the marker(s) associated therewith. In one embodiment, the rapid dissolve layer dissolves in, or is soluble in, saliva. As saliva is generally water and digestive enzymes in a fluid of mildly acidic pH, materials and compounds which dissolve therein or are degraded by the enzymes are suitable. The rapid dissolve layer should be biologically acceptable and capable of being formulated with the marker(s) of choice.

While the composition may only contain the marker-containing rapid dissolve layer, the present invention is not so limited and the composition may further include other layers or components. In one embodiment, the composition includes a first layer overlying the rapid dissolve layer. As such, the first layer may provide protection for the rapid dissolve layer, the marker, and for the composition as a whole, when the composition is not in use, or on a medication to be ingested. A protective first layer is useful when the composition is stored after manufacture, and used in combination with a medicament as needed. The first layer may also provide additional stability for the marker(s) contained in the rapid dissolve layer. For example, the first layer may protect the marker(s) from elements of the environment (e.g., humidity, air), desiccation, abrasion, and even provide for the incorporation of adventitious material. In another embodiment, the first layer is colored or has a colored-coating for purposes of visual identification. Specific coloration of the first layer helps to identify not only the composition itself, but also medications having the composition placed thereon. For example, the colored first layer will allow the user or caregiver to detect the presence of the composition on a medication thus preventing repeated placement of compositions on a single unit of the medication thereby reducing the potential for waste. For purposes of ingestion, the first layer should be a pharmaceutically acceptable material, In one embodiment the first layer is an edible film. Optionally, the first layer may further include a marker for monitoring and/or identification purposes.

In accordance with the principles of the present invention, the composition is placed on the medication prior to ingestion by the patient. To this end, and in one embodiment of the invention, an adhesive is included on a surface of the rapid dissolve layer to adhere it and the composition to the medication. In another embodiment, the adhesive is on a side of the rapid dissolve layer opposite the first layer, where included in the composition. In another embodiment, the adhesive may be integrated with the underlying layer of the composition for contact with the medication. For example, where the composition is a web or sheet, the adhesive may be incorporated into the bottom or lower most layer of the web or sheet which contacts the medication. The adhesive should be medically safe, or approved for ingestion, and present in the composition in an amount sufficient to secure the composition to the medication. Generally, where the composition is small, such as a dot or a microdot, small amounts of adhesive will suffice.

While the description of the composition above provides for a marker-containing rapid dissolve layer, a first layer, and/or an adhesive layer or component in the composition, the composition is not so limited and may further include other layers or components as desired. For example, the composition may include a release liner to cover or protect the adhesive when the composition is not being used. The release liner may generally be a backing layer removably applied over the adhesive and which peels off of the composition to expose the adhesive. The backing layer may be, for example, a tough plastic laminate or a reinforced plastic laminate which is resistant to tearing, but easily cut or severed with a knife or blade. Thus, the release liner allows the composition to be stored for long periods of time without suffering a loss of adhesive which might otherwise prevent proper application and/or prolonged adhesion of the composition to the medication. As the composition adhered to the release liner is intended for ingestion, the release liner should be a material that is non-toxic and/or pharmaceutically acceptable. In one embodiment, the release liner is a medical grade release liner. Any non-pharmaceutically accepted materials should not be present in toxic amounts as such materials may diffuse into the composition while the release liner is adhered thereto.

With one or more layers, in accordance with aspects of the present invention, the compositions are small, simple and easy to use. To this end, the composition may take a form adapted to be easily secured to a medication. For example, the composition may be in the form of a web, a strip, a sheet, a dot, or other form capable of being conveniently adhered to the medication. Where the composition is in the form of a continuous strip or sheet, the user may cut off or peel off from a backing layer a small piece of the strip or sheet for attaching to the medication. Alternatively, the composition may be in the form of a dot, such as a tape dot, a micro-sized dot, or a multilamelar dot, which individually may be adhered to the medication without the need for cutting or breaking from a bigger compositional piece. Further, such forms may have a release layer for storage and protection of the composition, as described above, and for ease of application of the composition to the medication as and when needed. The release liner may be part of a kis-cut surface such that a plurality of individual compositions are adhered to a single release liner surface in kis-cut fashion. Such kis-cut technology may be utilized in manufacturing any of the forms of the composition as described below.

The forms of the present composition described above may be made or fabricated by conventional methods known in the art. For example, a dot or micro-sized dot composition may be formed in a desired pattern, such as in series, onto a sheet, such as a microfilm sheet, which may then be processed to adhesively secure a removable backing layer. The dots may then be punched or at least partially severed from the sheet but without removal from the backing layer. The sheet is preferably divided into a series of rows or strips of individual dots, and made commercially available. In order to apply the dot to a medication, the user, such as a caregiver, simply removes the backing layer by hand or other method from a single dot and places the dot on a medication to be ingested by a patient. In one embodiment, the "forms" of the composition are prepared using kis-cut technology. It should be understood that the forms of the composition and method of application described above are by way of example only, and the present invention is not so limited.

The medication, or medicament, on which the composition is placed or adhered may be, for example, a solid form such as a pill, a tablet, a capsule, or a soft-gel capsule, suitable for ingestion. To this end, the composition is generally adhered to the medicament's surface and this combination, or medicament formulation, is provided to the patient for ingestion. Exposure to, or contact with, saliva causes the composition to release the marker(s) thereby effecting a visibly observable marker-induced property in the patient's oral and/or pharyngeal cavity. For example, the composition may be placed on an exposed surface of the medicament so that, upon ingestion, the medicament formulation contacts the tongue, buccal membrane, throat, and other areas of the oral and/or pharyngeal cavity thereby stimulating the excretion of saliva, and the release of the marker. The patient will thereafter be examined for detection of the marker(s).

The present invention contemplates failure to comply with a medication regimen by failing to ingest the medicament formulation. In this event, the location of this formulation would need to be discovered. To this end, the outermost, or overlying, layer of the composition, whether it is the first layer or the rapid dissolve layer, may be formulated such that it has friable properties and readily crumbles or pulverizes when subjected to friction or other pressure thereby releasing the marker(s) contained therein and staining the areas to which the marker comes into contact. For example, in the event that the patient refuses to ingest a "marked" medication, one on which the composition has been adhered, and instead places the formulation in his/her pocket in non-compliance with a medication regimen, a composition having an outer friable layer comprising a powdery solid marker will be useful. In this instance, the composition will generally crumble from the friction due to the contact with the inner lining of the patient's pockets, thereby releasing the marker and staining the pocket for later detection and determination of non-compliance by the patient. Similarly, one or more markers in a friable composition adhered to a medication may release the markers in an article, container, or other location, under similar forces, to provide an indication of the location of the "marked" medication.

Still further, the present invention contemplates the identity of a particular medication. For example, the composition may have an outer layer which is uniquely colored, such as the overlying first layer described earlier, to provide a means to identify specific medications which have been previously "marked" with the marker-containing composition. As a further example, the coloration in the layer may respond only to fluorescent light and thus be detectable only under fluorescence, and not visible to the naked eye under natural light. This is useful in the event the medication is stolen from its original location or source, such as a manufacturer's warehouse, a distribution center, a clinic, a hospital room, or a patient's home. For such a purpose, a clear, non-colored marker may be suitable.

As noted above, while the coloration of the oral and/or pharyngeal cavity is necessary for determining compliance, the detectable presence of the marker will not have a duration which outlasts the period between medication doses. Otherwise, a coloration may exist when medication has not been ingested in compliance with a designated regimen. Such false positive results would adversely affect the accuracy of compliance testing. Thus, in accordance with one aspect of the present invention, in order to ensure that the marker does not remain visible for a period of time longer than the pharmacological efficacy of the medication composition, a marker may be chosen wherein the duration of the marker in the human system is in cooperation with the half-life of the medication composition in the human system. In one embodiment, the marker has a detectable duration generally equal to the half-life of the medication. Similarly, multiple markers with various different durations may be used for different purposes, as discussed above.

The present invention also provides methods for monitoring the ingestion of a medication by a patient. This is useful for monitoring compliance in following a medication regimen. In this method, a patient is provided with an orally administrable medicament and a saliva-activated rapid dissolve layer including a marker. As described above, the rapid dissolve layer may be present in a "compositional" form and contain the marker(s) in an amount which is sufficient to cause a detectable marker-induced property, such as coloration of at least a portion of the oral and/or pharyngeal cavity of the patient. The rapid dissolve layer or composition is conveniently secured to the medication, generally by a caregiver, and the patient is allowed to ingest a scheduled dosage of the medication. Following ingestion, the patient and/or an article associated with the patient is examined for detection of the marker. Examination methods may differ depending upon the individual markers. For example, the patient's oral and/or pharyngeal cavity may be examined by visual inspection and observation using a suitable source of light. Where a combination of markers are used, lights of various wavelengths, color, and intensity may be used for detection of the markers. The observer, such as the caregiver, then determines the presence or absence of the marker-induced property, such as coloration, of the patient's oral and/or pharyngeal cavity.

Also, the specific portion of the oral and/or pharyngeal cavity being observed is the oral mucous membrane or buccal membrane. If the patient has actually ingested the composition and marker, whose detectable property is coloration, in accordance with the principles of the present invention, then the mucous membrane will be stained or colored with a color indicative of that specific marker. By periodically observing the mucous membrane of the oral and/or pharyngeal cavity for such coloration, according to a determined regimen, one can determine whether a patient is following a medication regimen. Thus, if no coloration is apparent upon observation of the oral and/or pharyngeal cavity, a caregiver can either require that the patient take the proper dosage of medication, or, if refusal persists, the caregiver can alter the method of medication delivery.

Also, as described above, the oral and/or pharyngeal cavity may be observed under any of a number of suitable lights, such as visible light, ultraviolet light, infra-red light, X-ray, or under a light which causes fluorescence at an optimal exciting wavelength. More particularly, in the method of the present invention, inspection may be carried out immediately following the time for scheduled ingestion by visually observing the oral and/or pharyngeal cavity of the patient for a color that is characteristically invoked by the colored marker. This observation may be carried out under natural light and with the naked eye. In the embodiment of the present invention using carmine dye, for example, a reddish coloration would appear on a mucous membrane in the oral and/or pharyngeal cavity of the patient. If a time lapse occurs between the time scheduled for ingestion and the time of actual observation, the visually apparent color caused by the marker may evanesce, leaving little or no apparent coloring of the oral and/or pharyngeal cavity. However, determination of ingestion of the medication and composition may still be obtained through fluorescence of the oral mucous membrane. In this embodiment of the method of the present invention, a fluorescent light may be used to evoke a visually apparent emission of a color which is characteristic of the marker. Using carmine red dye, the reddish-orange residue left by carmine red dye would be observed. In order to observe the carmine red marker residue by fluorescence, an optimal exciting light is directed into the oral and/or pharyngeal cavity of the patient. To fluoresce the residue of carmine red dye, as in the one embodiment of the present invention, the light is either a violet-blue light having a wavelength of about 436 nanometers, or a blue light having a wavelength in a range of from about 450 nm to about 490 nm might be used. Moreover, as the marker evanesces, the light might be adjusted to a desirable wavelength for maximum detection of the coloration or discoloration. Therefore, an optimal exciting light in the violet-blue to blue range of about 430 nm to 490 nm may be used. Of course, light of different appropriate wavelengths may also be used, depending upon the characteristics of the marker.

The patient's oral and/or pharyngeal cavity is not the only area which should be examined. The marker(s) may have been released onto articles associated with the patient and/or other persons tending to the patient. In one embodiment, an article, such as clothing is examined. In another embodiment, a body part is examined. Particularly, the pockets of the clothing or the hands of a person may be visually inspected for determining location of a medicament, and for determining that the medicament was not ingested. As such, the marker-containing rapid dissolve layer or compositions of present invention may be used for labelling medications for inventory purposes, and as well as for labelling any ingestible item for subsequent tracking of the item.

While the present invention has been illustrated by the description of various embodiments thereof, and while these embodiments have been described in considerable detail, additional advantages and modifications will readily appear to those skilled in the art.

## Claims

1. A composition comprising at least one detectable marker formulated with a rapid dissolve layer, the composition adapted to be placed on a medicament.

2. The composition of claim 1 in a form selected from the group consisting of a web, a sheet, a strip, a dot, a microdot, a tape dot, and a multilamelar dot.

3. The composition of either claim 1 or claim 2 further comprising a first layer overlying the rapid dissolve layer.

4. The composition of claim 3 wherein the first layer is colored to render the composition visible.

5. The composition of either claim 3 or claim 4 wherein the first layer is a pharmaceutically acceptable film.

6. The composition of any preceding claim wherein the marker is released upon exposure of the rapid dissolve layer to saliva.

7. The composition of any preceding claim wherein the rapid dissolve layer dissolves in saliva to release the at least one marker.

8. The composition of any preceding claim wherein the rapid dissolve layer further comprises a mucoadhesive attached to at least one marker.

9. The composition of any preceding claim wherein the at least one marker is selected from the group consisting of a dye, a fluorescent compound, a visually observable solid, and a combination thereof.

10. The composition of any one of claims 1 to 8 wherein the at least one marker is clear.

11. The composition of any one of claims 1 to 8 wherein the at least one marker is colored.

12. The composition of any one of claims 1 to 8 wherein the at least one marker is selected from the group consisting of indigo carmine, carmine red, methylene blue, tartrazine, laccaic acid, beta-carotene, FD&C blue 1, FD&C blue 2, FD&C green 3, FD&C red 3, FD&C red 40, FD&C yellow 6, and riboflavin.

13. The composition of any preceding claim further comprising a plurality of markers, each marker in an amount sufficient to cause coloration of a portion of an oral and/or pharyngeal cavity.

14. The composition of claim 13 wherein one of the markers causes a detectable coloration different from another of said markers.

15. The composition of claim 13 wherein one of the markers causes a coloration detectable with visible light and another of the markers causes coloration detectable with a light which causes fluorescence.

16. The composition of claim 13 wherein the plurality of markers are detectable with a light which causes fluorescence, one of said markers causing a fluorescent coloration different from the fluorescent coloration caused by the other markers.

17. The composition of any preceding claim further comprising an adhesive on the rapid dissolve layer to adhere the composition to the medicament.

18. The composition of claim 17 wherein the adhesive is a pharmaceutically acceptable material.

19. The composition of either claim 17 or claim 18 further comprising a release liner adhered to the adhesive on a surface opposing the rapid dissolve layer.

20. The composition of claim 19 wherein the release liner is medical grade.

21. The composition of either claim 19 or claim 20 wherein the release liner is part of a kis-cut surface.

22. A medicament formulation comprising an ingestible medicament and a composition of any preceding claim thereon.

23. A medicament formulation comprising an ingestible medicament and a composition of any one of claims 1 to 16 wherein the rapid dissolve layer is adhered to the medicament with an adhesive.

24. The formulation of claim 23 comprising wherein the adhesive is a pharmaceutically acceptable material.

25. A medicament formulation comprising an ingestible medicament and a composition of any one of Claim 1 to 16 wherein the composition is adhered to a surface of the medicament.

26. The formulation of claim 25 wherein the composition further comprises a pharmaceutically acceptable adhesive for adhering the composition to the medicament.

27. The formulation of either claim 25 or claim 26 wherein the composition is adhered to the medicament after manufacture of the medicament.

28. The formulation of either claim 25 or claim 26 wherein the composition is adhered to the medicament before packaging of the medicament.

29. The formulation of either claim 25 or claim 26 wherein the composition is adhered to the medicament after packaging of the medicament.

30. The formulation of either claim 25 or claim 26 wherein the composition is adhered to the medicament at the point of oral administration of the medicament.

31. The formulation of any one of Claims 22 to 30 wherein the medicament is a solid selected from the group consisting of a pill, a tablet, a capsule, and a soft-gel capsule.

32. The formulation of any one of claims 22 to 31 wherein the half-life of the marker in a mammalian system is comparable to the half-life of the medicament in the mammalian system.

33. A kit for monitoring patient compliance with dosing of a medicament, the kit comprising a composition as claimed in any one of claims 1 to 21.

34. The kit of claim 33 further comprising an orally administrable medicament.

35. A kit for monitoring patient compliance with dosing of a medicament, the kit comprising a medicament as claimed in any one of claims 22 to 32.

36. A method of tracking a medicament comprising providing a medicament with a compliance marker formulated with a rapid dissolve layer and examining for a marker-induced property to track the medicament.

37. The method of claim 36 further comprising examining an article for a marker-induced property.

38. The method of claim 37 wherein the article is clothing.

39. The method of claim 37 wherein the article is a body part.

40. The method of any one of claims 36 to 39 wherein the rapid dissolve layer is adhered to the medicament after manufacture of the medicament.

41. The method of any one of claims 36 to 39 wherein the rapid dissolve layer is adhered to the medicament before packaging of the medicament.

42. The method of any one of claims 36 to 41 wherein examining comprises visually inspecting with a light selected from the group consisting of visible light, ultraviolet light, infra red light, X-ray, and fluorescent light.

43. The method of any one of claims 36 to 42 wherein the method monitors compliance with a medicament regimen.
